## Europäisches Patentamt

## European Patent Office

## Office européen des brevets

(19)

(11) Veröffentlichungsnummer: **0 026 796**
**A1**

# EUROPÄISCHE PATENTANMELDUNG

(12)

(21) Anmeldenummer: **79104275.7**

(22) Anmeldetag: **02.11.79**

(51) Int. Cl.³: **A 61 B 5/05**

(30) Priorität: **08.10.79 DE 2940724**

(43) Veröffentlichungstag der Anmeldung: **15.04.81**
**Patentblatt 81/15**

(84) Benannte Vertragsstaaten: **AT BE CH FR GB IT LU NL SE**

(71) Anmelder: **Rietz, Erhard, Rathsbergstrasse 25, D-8500 Nürnberg (DE)**

(72) Erfinder: **Leupold, Karl-Heinz, Dr.h.c., Gudrunstrasse 41, D-8500 Nürnberg (DE)**

(74) Vertreter: **Göbel, Matthias, Pruppacher Hauptstrasse 5-7, D-8501 Pyrbaum-Pruppach (DE)**

(54) **Vorrichtung zum Messen der vegetativen Reaktionslage des menschlichen Organismusses.**

(57) Bei der Vorrichtung zum Messen der vegetativen Reaktionslage des menschlichen Organismuses sind zwei mittels Schaltglieder wahlweise mit einer im Vorrichtungsgehäuse (1) angeordneten Stromquelle und einem Strommeßgerät in Reihe schaltbare oder an eine spannungsveränderliche Einrichtung anlegbare, durch Rohr- oder Profilabschnitte aus metallischem Werkstoff gebildete Berührungselektroden (2) vorgesehen. Die Berührungselektroden (2) sind mit einem Ende am Vorrichtungsgehäuse (1) festgelegt, während die freien Enden der Berührungselektroden (2) bis mindestens annähernd zur Aufstellfläche des Vorrichtungsgehäuses (1) sich erstreckende Stützkörper (5) tragen.

Fig. 1

# M. GÖBEL
## PATENT- U. ZIV.-INGENIEUR

**0026796**

**8501 PYRBAUM-PRUPPACH**
PRUPPACHER HAUPTSTRASSE 5-7
TELEFON 091802 / 675

BANKKONTEN:
VOLKSBANK NÜRNBERG 45 2 33 BLZ 760 900 00
COMMERZBANK NÜRNBERG 8 300 907 BLZ 760 400 61

- 1 -

Firma Erhard Rietz, 8500 Nürnberg

Vorrichtung zum Messen der vegetativen Reaktionslage des menschlichen Organismusses

Die Erfindung betrifft eine Vorrichtung zum Messen der vegetativen Reaktionslage des menschlichen Organismusses unter Verwendung von zwei unabhängigen Berührungselektroden, die mittels Schaltglieder wahlweise mit einer im Vorrichtungsgehäuse angeordneten Stromquelle und einem Strommeßgerät in Reihe geschaltet oder an eine spannungsveränderliche Einrichtung anlegbar sind.

Mit den bekannten Vorrichtungen dieser Art sind Leitwertmessungen des menschlichen Körpers, z.B. zwischen Hand-Hand oder Hand-Fuß bzw. Fuß-Fuß möglich und entsprechend der Meßwerte elektrische Auf- und Abladungen durchführbar. Die Berührungselektroden sind hierzu über flexible elektrische

-/

Leiter mit den im Vorrichtungsgehäuse untergebrachten Einrichtungen in Verbindung gebracht. Abgesehen davon, daß die so erzielte ortsbewegliche Ausbildung der Berührungselektroden die Handhabung, insbesondere den Transport der Vorrichtungen erschwert, sind Messungen bei denen ein Erfassen der Berührungselektroden nicht erfolgt, z.B. bei der Fuß-Fuß-Messung nur schwierig vorzunehmen. Die Berührungselektroden werden hierzu in der Regel auf die Bodenfläche gelegt, was desöfteren durch unterschiedliche Isolationswerte in der Bodenfläche zu Fehlmessungen Anlaß gibt.

Die Erfindung hat die Schaffung einer Vorrichtung der vorerwähnten Art zur Aufgabe, die unter Ausschaltung der den bekannten Vorrichtungen anhaftenden Nachteile, einfach zu handhaben ist.

Der Erfindung gemäß ist diese Aufgabe dadurch gelöst, daß die Berührungselektroden durch Rohr- oder Profilabschnitte aus metallischem Werkstoff gebildet und mit einem Ende am Vorrichtungsgehäuse festgelegt sind und daß die freien Enden der Berührungselektroden Stützkörper fest tragen, die sich bis mindestens annähernd zur Aufstellfläche des Vorrichtungsgehäuses erstrecken. Bevorzugt greifen die Berührungselektroden zum sicheren Erfassen diametral am Vorrichtungsgehäuse

-/

an. Auf diese Weise bildet die Vorrichtung insgesamt eine Baueinheit, die als solche in Benutzung nehmbar ist. Außerdem läßt die gehäusefeste Anordnung der Berührungselektroden gleichermaßen das Erfassen derselben und die behinderungsfreie Verwendung als Stützen, z.B. bei Fuß-Messungen zu. Die auf den freien Enden aufgebrachten Stützkörper können dabei beliebig gestaltet sein. Zweckmäßig sind die Stützkörper jedoch als Scheibenkörper oder Formkörper ausgeführt, die an dem der Aufstellfläche zugewandten Ende eine ebene Stützfläche aufweisen.

In Ausgestaltung der Vorrichtung ist vorgesehen, die gehäuseseitigen Enden der Berührungselektroden entweder mittels Schraubverbindung, Nietung oder Klemmglieder bzw. durch Kleben, Vulkanisation oder Schweißen mit dem Vorrichtungsgehäuse fest zu verbinden.

Es entspricht der Erfindung, daß die Berührungselektroden aus einem metallischen Werkstoff mit großer elektrischer Leitfähigkeit, z.B. Kupfer oder Kupferlegierung, beispielsweise Messing gebildet sind. Es ist auch denkbar, die Berührungselektroden außen glatt oder mindestens über eine Teillänge mit Griffflächen zu versehen, die gegebenenfalls den Übergangswiderstand zwischen Haut und Berührungselektroden ver-

-/

kleinern helfen bzw. stabilisieren.

Schließlich ist noch vorgesehen, das Vorrichtungsgehäuse mit einer, z.B. über eine Gehäusekante greifenden Gehäuseöffnung frei zugänglichen Aufnahmekammer für eine Stromquelle zu versehen, die durch einen am Vorrichtungsgehäuse verrastbaren Deckelteil verschließbar ist. Der Deckelteil kann winkelförmig ausgebildet und mittels im Eckbereich desselben innen angeformten Nuten auf leistenartigen Vorsprüngen von zwei einander gegenüberliegenden Begrenzungsflächen der Aufnahmekammer aufsteckbar sein, während eine an einem Schenkel des Deckelteils angeordnete Lasche in eine Ausnehmung einer quer zu den Begrenzungsflächen sich erstreckenden Begrenzungsfläche der Aufnahmekammer eingreift und dort verrastet.

Weitere Maßnahmen zur Verbesserung der Standfestigkeit der Vorrichtung können ferner noch dadurch erzielt sein, daß das Vorrichtungsgehäuse in der Bodenfläche außen diese gering überragende Auflagen aus einem flexiblen oder federnd elastischen Werkstoff, z.B. einem Gummiwerkstoff, aufweist.

Wie die Erfindung ausgeführt sein kann, zeigt das in der Zeichnung dargestellte Ausführungsbeispiel. Hierin bedeuten:

-/

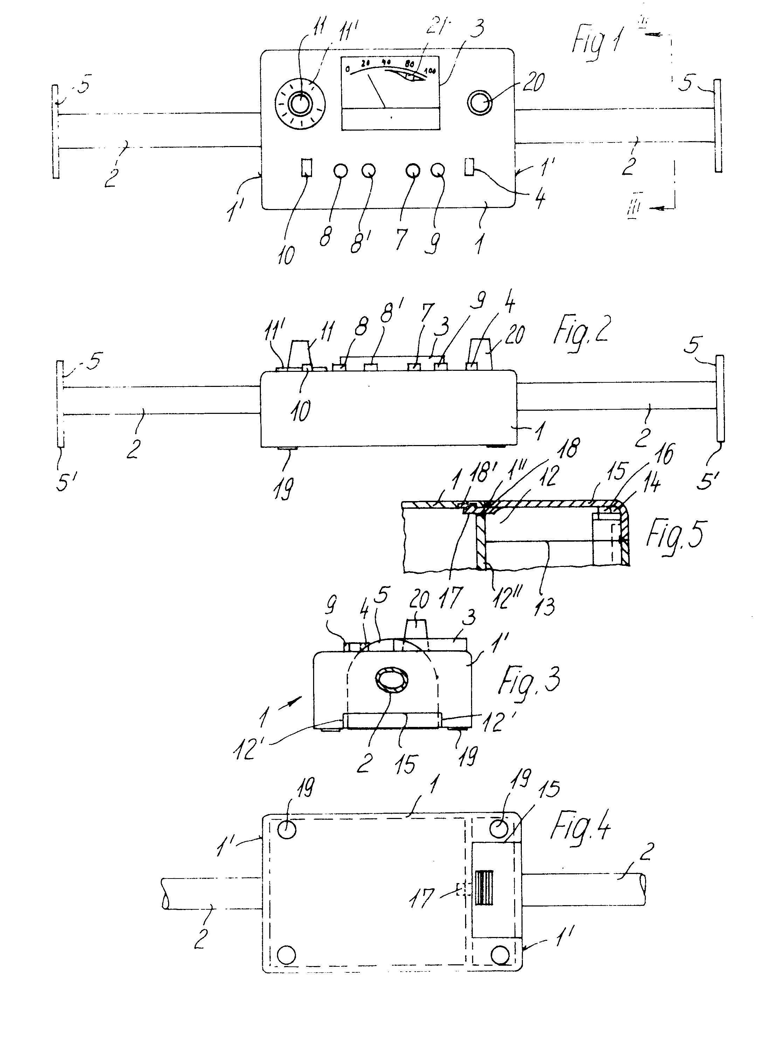

Fig. 1     eine Vorrichtung in Draufsicht,

Fig. 2     eine Vorrichtung in Vorderansicht,

Fig. 3     eine Vorrichtung im Schnitt nach der Linie III-III
           der Fig. 1,

Fig. 4     ein Teilstück einer Vorrichtung in Unteransicht und

Fig. 5     einen Teilschnitt einer Vorrichtung, vergrößert.


In den Figuren ist mit 1 ein, z.B. aus Kunststoffmaterialien hergestelltes, kastenförmiges Vorrichtungsgehäuse bezeichnet, das der Unterbringung einer Stromquelle und einer Einrichtung (nicht gezeigt) zur Erzeugung niederfrequenter, veränderlicher und in der Polarität umkehrbarer Spannungen dient. Am Vorrichtungsgehäuse 1 sind an zwei einander gegenüberliegenden Seitenwänden 1' je eine rohrförmige Berührungselektrode 2 aus einer Kupferlegierung, z.B. Messing, festgelegt. Außerdem nimmt das Vorrichtungsgehäuse 1 ein elektrisches Strommeßgerät 3 auf, das mittels eines Schalters 9 mit den beiden Berührungselektroden 2 und der Stromquelle zu Leitwertmessungen verbindbar bzw. über einen Schalter 4 und einem Betätigungsknopf 20 für einen Drehwiderstand mit der Stromquelle zu Eichzwecken in Reihe legbar ist. Bei Erfassen der beiden Berührungselektroden 2, z.B. mit den Händen des Benutzers, sind Leitwertmessungen der oberen Region des Körpers möglich.

-/

Die Berührungselektroden 2 tragen an ihren freien Enden Stützkörper 5, die beim Ausführungsbeispiel scheibenförmig aus Kunststoff hergestellt sind und sich mindestens annähernd bis zur Aufstellfläche des Vorrichtungsgehäuses 1 erstrecken. Die Stützkörper 5 sind an den der Aufstellfläche zugewandten Enden mit ebenen Stützflächen 5' versehen. Die Stützkörper 5 haben die Aufgabe bei Belastungen der Berührungselektroden 2, z.B. Aufstellen der Füße zu Leitwertmessungen, durch Anlage an der Aufstellfläche, die Berührungselektroden frei von störenden Abbiegungen zu halten. Weit nimmt das Vorrichtungsgehäuse 1 einen Drucktastenschalter 7 für Ab- und Aufladen sowie Drucktastenschalter 8, 8' zum Anlegen zugehöriger negativer oder positiver Spannungen an den Berührungselektroden 2 sowie einen Schalter 10 als Startschalter auf. Mit 11 ist ein Drehknopf mit Skala 11' für ein Potentiometer zum Einregeln der Intensität der an den Berührungselektroden 2 anliegenden Spannung bezeichnet.

Das Vorrichtungsgehäuse 1 weist weiter eine Aufnahmekammer 12 für eine Stromquelle, z.B. eine Batterie auf, die über eine sich über eine Kante 1'' erstreckende und durch einen Deckelteil 15 verschließbare Gehäuseöffnung 13 zum Einbringen oder Herausnehmen der Stromquelle zugänglich ist. Der Deckelteil 15 weist, wie insbesondere in Fig. 5 ersichtlich, Nuten 14 auf,

-/.

die auf leistenartigen Vorsprüngen 16 der seitlichen Begrenzungsflächen 12' der Aufnahmekammer 12 zur Fixierung des Deckelteils 15 aufsteckbar sind. Außerdem ist am Deckelteil 15 eine Lasche 17 angeformt, die in eine Ausnehmung 18 der Begrenzungsfläche 12'' eintaucht und an den inneren Randflächen der Ausnehmung 17 oder einer Einziehung 18' (Fig. 5) verrastet.

Ferner weist das Vorrichtungsgehäuse 1 in der Bodenfläche außen Auflagen 19, z.B. aus Gummiwerkstoff auf, die für eine rutschfeste Aufstellung der Vorrichtung auf Aufstellflächen sorgen.

Die Anzeigebereiche am Strommeßgerät 7 können durch der Skala des Strommeßgerätes 7 zugeordnete Farbmarkierungen 21 verdeutlicht bzw. ersetzt sein.

# M. GÖBEL
## PATENT- U. ZIV.-INGENIEUR

**8501 PYRBAUM-PRUPPACH**
PRUPPACHER HAUPTSTRASSE 5-7
TELEFON 091802 / 675

BANKKONTEN:
VOLKSBANK NÜRNBERG 45 2 33 BLZ 760 900 00
COMMERZBANK NÜRNBERG 8 300 907 BLZ 760 400 61

0026796

29. Okt. 1979

Firma Erhard Rietz, 8500 Nürnberg

Patentansprüche

1. Vorrichtung zum Messen der vegetativen Reaktionslage des menschlichen Organismusses unter Verwendung von zwei unabhängigen Berührungselektroden, die mittels Schaltglieder wahlweise mit einer im Vorrichtungsgehäuse angeordneten Stromquelle und einem Strommeßgerät in Reihe geschaltet oder an eine spannungsveränderliche Einrichtung anlegbar sind, dadurch gekennzeichnet, daß die Berührungselektroden (2) durch Rohr- oder Profilabschnitte aus metallischem Werkstoff gebildet und mit einem Ende am Vorrichtungsgehäuse (1) festgelegt sind und daß die freien Enden der Berührungselektroden (2) Stützkörper (5) fest tragen, die sich bis mindestens annähernd zur Aufstellfläche des Vorrichtungsgehäuses (1) erstrecken.

2. Vorrichtung nach Anspruch 1, dadurch gekennzeichnet, daß die Berührungselektroden (2) diametral am Vorrichtungsgehäuse (1) festgelegt sind.

-/2

3. Vorrichtung nach Anspruch 1, dadurch gekennzeichnet, daß die gehäuseseitigen Enden der Berührungselektroden (2) mittels Schraubverbindung, Nietung oder durch Klemmglieder am Vorrichtungsgehäuse (1) festgelegt sind.

4. Vorrichtung nach Anspruch 1, dadurch gekennzeichnet, daß die gehäuseseitigen Enden der Berührungselektroden (2) durch Kleben, Vulkanisation oder Schweißen mit dem Vorrichtungsgehäuse (1) fest verbunden sind.

5. Vorrichtung nach Anspruch 1, dadurch gekennzeichnet, daß die Stützkörper (5) durch Scheibenkörper oder Formkörper gebildet sind und an dem der Aufstellfläche zugewandten Ende eine ebene Stützfläche (5') aufweisen.

6. Vorrichtung nach Anspruch 1, dadurch gekennzeichnet, daß die Berührungselektroden (2) aus einem metallischen Werkstoff mit großer elektrischer Leitfähigkeit, z.B. Kupfer oder Kupferlegierung gebildet sind.

7. Vorrichtung nach einem oder mehreren der vorhergehenden Ansprüche, dadurch gekennzeichnet, daß die Berührungselektroden (2) außen glatt oder mindestens über eine Teillänge Griffflächen aufweisen.

-/3

8. Vorrichtung nach Anspruch 1, dadurch gekennzeichnet, daß das Vorrichtungsgehäuse (1) eine über eine Gehäusekante (1') sich erstreckende Gehäuseöffnung (13) frei zugängliche Aufnahmekammer (12) für eine Stromquelle aufweist, die durch einen am Vorrichtungsgehäuse (1) verrastbaren Deckelteil (15) verschließbar ist.

9. Vorrichtung nach Anspruch 8, dadurch gekennzeichnet, daß der Deckelteil (15) winkelförmig ausgebildet und mittels im Eckbereich desselben innen angeformte Nuten (14) auf leistenartigen Vorsprüngen (16) an zwei einander gegenüberliegende Begrenzungsflächen (12') der Aufnahmekammer (12) aufsteckbar ist und mittels einer an einem Schenkel des Deckelteils (15) angeordneten Lasche (17) in einer Ausnehmung (18) einer quer zu den Begrenzungsflächen (12') sich erstreckenden Begrenzungsfläche (12'') der Aufnahmekammer (12) eingreift und verrastet ist.

10. Vorrichtung nach Anspruch 1, dadurch gekennzeichnet, daß das Vorrichtungsgehäuse (1) in der Bodenfläche diese außen überragende Auflagen (19) aus einem flexiblen oder federnd elastischen Werkstoff aufweist.

0026796

Fig 1

Fig. 2

Fig.5

Fig. 3

Fig.4

Europäisches
Patentamt

**EUROPÄISCHER RECHERCHENBERICHT**

0026796
Nummer der Anmeldung

EP 79104275.7

| | EINSCHLÄGIGE DOKUMENTE | | | KLASSIFIKATION DER ANMELDUNG (Int. Cl.³) |
|---|---|---|---|---|
| Kategorie | Kennzeichnung des Dokuments mit Angabe, soweit erforderlich, der maßgeblichen Teile | | betrifft Anspruch | |
| A | <u>DE - A - 2 018 482</u> (H. SCHÄFER)<br>+ Seite 4, 2. Absatz +<br>-- | | 1,6 | A 61 B 5/05 |
| A | <u>US - A - 4 052 978</u> (A. EUGENIO)<br>+ Gesamt +<br>-- | | 1,6,7 | |
| A | <u>US - A - 3 727 604</u> (T.W. SIDWELL, G.F. REESE)<br>+ Gesamt +<br>---- | | 1,6 | |

**RECHERCHIERTE SACHGEBIETE (Int. Cl.³)**

A 61 B 5/00

A 61 N 1/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 09-01-1981 | LUDWIG |

EPA form 1503.1  06.78